Europäisches Patentamt

European Patent Office

Office européen des brevets

(19)

(11) Publication number: **0 163 811 B1**

# EUROPEAN PATENT SPECIFICATION

(12)

(45) Date of publication of patent specification: **28.08.91**   (51) Int. Cl.⁵: **A61M  1/28**

(21) Application number: **85101915.8**

(22) Date of filing: **21.02.85**

(54) Dialysis device.

(30) Priority: **06.03.84 IT 1990584**

(43) Date of publication of application:
**11.12.85 Bulletin  85/50**

(45) Publication of the grant of the patent:
**28.08.91 Bulletin  91/35**

(84) Designated Contracting States:
**AT BE CH DE FR GB LI NL SE**

(56) References cited:
**WO-A-83/03538**
**FR-A- 2 202 252**
**FR-A- 2 222 105**
**US-A- 4 306 976**

(73) Proprietor: **BIEFFE MEDITAL S.p.A.**
**Via Nuova Provinciale**
**I-23034 Grosotto(Sondrio)(IT)**

(72) Inventor: **Baldini, Luciano**
**Via Industria 38**
**I-23037 Tirano (SO)(IT)**

(74) Representative: **Incollingo, Italo**
**Piazzale Lavater, 3**
**I-20129 Milano(IT)**

EP 0 163 811 B1

## Description

The present invention relates to a device for continuos ambulatory peritoneal dialysis, comprising: a first bag, initally to be filled with dialytic fluid; a second bag, initially to be empty; output tubes connected to each of the bags; a first y-junction connecting said output tubes to a common tube; a first coupling element on the free end of the common tube comprising a cap and a needle; a second y-junction comprising a means for coupling with the first coupling element; a catheter; and a further tube connecting the catheter and the second y-junction.

In prior patents, particularly in the U.S. Patent No. 4,306,976 Applicant has described a method and a device for carrying out continuous ambulatory peritoneal dialysis(CAP),in which two bags are employed, one bag containing fresh dialytic liquid, and the other bag being empty and destined to the collection of the drained liquid.

Both the tube feeding the liquid from the filled bag and the tube of input to the empty bag are connected, at one of their ends, to a first y-shaped junction from which starts a third tube that is provided at its remote end with a sterile needle to be inserted into a second y-junction having, within each one of its branches, two membranes forming a chamber containing a sterilizing or antiseptic liquid.

The common output channel of this second junction is connected to the peritoneal catheter.

To operate a device of the above type it is necessary:

1) to take away the protecting cap from the needle with consequent risks of contact contamination and of possible psychological discomfort of the patient at the sight of the needle;

2) exert a certain pressure to insert said needle, in one of the antiseptic liquid containing chambers, by perforating the two membranes, with difficulty in correctly positioning the needle, especially when old and/or handicaped persons (f.i. blinds) have to actuate said perforation.

This system has obtained a large success based on its decisive advantages over the conventional methods of peritoneal dialysis using only one bag.

By continuing the research in this field Applicant has succeeded in imparting further improvements to said double bag system and device. Indeed one of the objects of the present invention is to provide a system for the double bag peritoneal dialysis, from which all risks of contamination of the needle and of the dialytic liquid are eliminated.

An other object of the invention is to provide a system of the above mentioned kind, in which the perforating needle is guided to a feed point of the membrane within the y-junction branches, allowing thus a much easier perforation and a longer life of the membranes and, therefore, of the relevant y-junctions.

A further object of the invention is to provide a system which besides being exempt of needle contamination rise and showing high perforation facility and long life of the membrane within the y-junction, is moreover particularly acceptable to the patients under the psychological point of view.

These and other objects are reached with the device according to the invention which is characterized according to the inventive clause of the claims. Accordingly

- to the dialytic liquid adducting needle are associated means of sterile protection and means for mechanical coupling; and
- to each one of the two branches of the y-junction containing the relevant disinfection chamber delimited by the two membranes, are associated removable covering means, means to guide the needle towards a substantially fixed zone of the membrane and means of mechanical engagement which are complementary to said mechanical coupling means associated to the dialytic liquid adducting needle.

According to an advantageus feature of the invention said extensible and retractable covering element hyde to the patient sight the whole needle when the complementary means on the y-junction are not engaged, respectively hyde the needle portion remaining outside said junction when same means are engaged.

The guide and covering means of the free ends of each branch of the y-junction, consist of:

- an annular portion restricting the inner diameter of said ends; and
- wings extending outwards to engage said covering means of each branch of the y-shaped junction in rest conditions, respectively the threaded retractable cap associated to the needle ends in operative conditions.

It is noted that WO 83/03538 describes a set for connection to a catheter comprising a first connector made of an UV transparent material,to which a bellows may be associated,when said first connector is screw-threated with a second connector the junction,the diaphragm and the spike thereto associated are irratiated for antibacterial effect prior to the spike penetrating said diaphragm.

The various features and advantages of the invention will better appear from the description of the preferred, not limiting embodiment shown in the attached drawing in which:

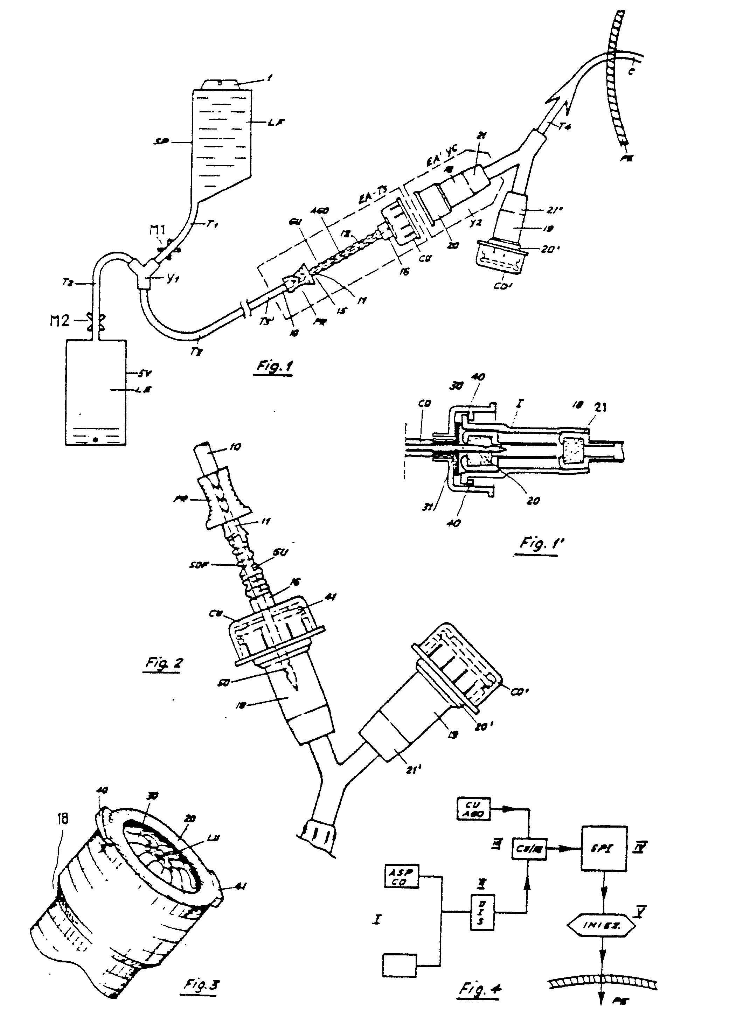

- Fig. 1 is a schematic partial front view of the whole dialysis apparatus incorporating the device according to the invention (generally indicated with reference A) in not-operative conditions;

- Fig. 1' is a schematic partially cross-sectioned view on enlarged scale of the right hand portion of A, in not-operative conditions;
- Fig. 2 is a schematic partial cross-section of the device according to the invention, on a slightly enlarged scale and in operative conditions;
- Fig. 3 is a perspective view of the surface guiding the needle into the disinfection chamber; and
- Fig. 4 is a block scheme illustrating the operative steps in using the device according to the invention.

Referring to figure 1 there are indicated with: SP the bag filled with the fresh dialytic fluid LS; SV the empty bag destined to receive the dialytic fluid exhausted at the end of the peritoneal dialysis; and $T_1$ and $T_2$ the small tubes of adduction of the fresh fluid from SP, respectively of return of the exhausted liquid LE to the initially empty bag SV. To each tube is associated a defluxion or flow control valve (f.i. closing keys or clamps) $M_1$ resp. $M_2$.

The ends of $T_1$ and $T_2$ remote from the ends connected to the bags are introduced in two branches of a first y-shaped junction from which outgoes a common tube $T_3$ at the free end of which is associated a first element EA-$T_3$ (within the rectangular dotted line at left hand) to be connected to a second element EA'-YC (within the rectangular dotted line at right hand) of a y-shaped junction whose branch in common goes in a small tube T4 of the catheter C already inserted into the patient peritoneum PE.

According to the invention the first element EA-$T_3$ shows a rigid piece PR which shows the end 10 firmly secured to the end $T'_3$ of tube $T_3$ and the other end 11 rigidly fixed to the needle AGO. In other words the rigid element PR works as needle-carrier and as rigid connection of tube $T_3$ to the needle end forced in said piece PR. According to the feature of the invention the needle mayor portion 12 abutting from 11 is covered by a sterile retractable sheath GU which extends substantially over the needle total length. Said sheath is fixed, on one side, to a protuberance 15 of element PR and, on the other side, to a projection 16 of a cap CU which is characteristically located at the needle free terminal portion, and projects slightly outside said portion.

Said cap CU is innerly threaded and is integral with the protuberance 16 having an inner diameter slightly higher than the needle outer diameter.

The other element EA'-YC comprises two branches 18 and 19 of the second y-shaped junction $Y_2$.

Each element 18 and 19 contains two membranes 20-21 respectively 20'-21' which form the disinfection chambers filled of a sterilizing liquid.

According to the invention, the input of each branch 18, 19 consists of a thin anular member 30 having a structure suitable to guide the needle into its inner free hollow surface 31 which is in front of a pre-determined zone preferably provided with an indentation area I formed in each membrane 20 respectively 20' (Fig. 1').

In not-operative conditions the top of each branch 18, 19 of the second y-junction $Y_2$ is protected by a removable cover CO which is screwd on the head of 18 resp. 19 by means of two small radial tongues 40 and 41 projecting outwardly from the outer surface of said elements 18, 19. Said rest position corresponding the inoperative conditions of the device of the invention, is shown in Fig. 1 and is characterized in that:

- needle AGO is entirely covered by sheath GU, the inwardly projecting extension 16 of CU covers substantially the needle point and the cap CU projects outwardly said needle point. According to an advantageous feature of the invention, in rest conditions the patient who has to set about his using this dialysis device does practically not see said needle (covered by the sterile sheath GU) and therefore does not undergo psychological discomfort;
- on the side EA'-YC (associated to the catheter) the two branches 18 and 19 including the disinfection chambers are normally closed by a cover or cap CO, resp. CO' screwd on the tongues 40 and 41.

To go now over to the true operative phase (see also Fig. 1' and the block diagramm of Fig. 4) the patient carries out following steps:

I Takes the protective cover CO (or CO') away from the top of one of the two branches 18 or 19 of the second y-junction $Y_2$ associated to the catheter C.

II Disinfects the upper portion of the head of f.i. cap 18 (previously freed of cover CO).

III Screws CU projecting on the expansion 4o and 41 of 18 (with no cover CO), said cap CU being located at the normally free end of the needle AGO and projecting outwardly from same end.

IV BY exerting a force on the rigid body PR, pushes the needle point towards membrane 20, same needle point being now guided by the inner anular portion 30 of 18 toward a same zone preferably provided with the indentation area I of membrane 20.

V The needle AGO is thus kept in operative position held fast in particular by the hole that same needle point has made in the membranes 20 and 21 and also by the central hole LU of the guiding anular portion 30 (Fig. 3). After a few minutes the needle portion penetrated in the disinfection chamber defined by the two membranes 20 and 21 and filled of antiseptic liquid, is innerly flown by the fresh dialytic liquid coming from the filled bag SP, after that the patient has opened clamp $M_1$. The above mentioned operations and their succession are indicated in the block diagramm of Fig. 4, under the reference: 1) ASP-CO (removal of cap CO); 2) DIS, disinfection of the top of the head of branch 18 (from which cover CO has been removed); 3) CU/18 screwing of cap CU on branch 18; 4) SPI, pushing the needle point toward membrane 20 and guiding same by means of the anular inner portion 30 of 18 towards the zone provided with indentation area I of membrane 20.

At the end of the dialysis the patient will close clamp $M_1$ and will open $M_2$ allowing thus the exhausted fluid to flow into the initially empty bag SV.

Advantageously either during the permanence phase of the dialytic fluid within the peritoneum, or during the exhausted liquid extraction the patient has the possibility of closing also $M_2$, exerting a minimum force on the rigid piece PR to extract the needle projecting portion from the membranes 20, 21, unscrewing the cap CU from protuberances 40, 41 (on 18 or 19) and screwing the cover CO (or CO') on same tongues or wings 40, 41 whereby the device is brought back to the initial inoperative condition.

The sheath GU extending between the end of the pushing rigid piece PR and the re-entrant end 16 of cap CU has plastomeric or elastomeric characteristics to retract as bellows SOF (Fig. 2) when the patient pushes PR to cause the penetration of the needle into the membranes 20 and 21. Said sheath GU is advantageously a simple tube made of thermoplastic material (with or without plasticizers) and/or with elastomeric characteristics whereby it exerts pratically no resistance when it passes from its entirely extended state of Fig. 1 to its shortened and retracted (bellows-like) state of Fig. 2.

Among the advantages of the invention we limit ourself to mention some of the very important ones such as:

1) Thanks to the sterile retractable sheath GU, contamination of the needle and thus of the input dialytic liquid is by no means possible, even in correspondence of wrong manoeuvring of the device by the patient.

2) The use of the needle is now simplified because the zone to perforate is pre-determined (in corrispondence of indentation area I) and there is no more need to tentatively search it as in the conventional system.

3) The perforation is easy as the needle is guided, perpendicularly to the membrane rubber surface to be perforated.

4) The perforation is even easier as it takes place always in a same point.

5) Longer life of the second y-junction because of the perforations in a same zone.

6) Better psychological acceptance by the patient of this "hydden needle system" as the needle is covered by GU.

7) Possibility of use of such a system also by handicaped patient, f.i. blinds (occurring rather frequently in this therapy type).

## Claims

1. A device for continuous ambulatory peritoneal dialysis. comprising:

    1. a first bag, (SP) initally to be filled with dialytic fluid (LF);

    2. a second bag, initially to be empty (SV);

    3. output tubes $(T_1-T_2)$ connected to each of the bags;

    4. a first y-junction $(Y_1)$ connecting said output tubes to a common tube $(T_3)$;

    5. a first coupling element $(EA-T_3)$ on the free end of the common tube $(T_3)$ comprising a cap (CU) and a needle (AGO);

    6. a second y-junction $(Y_2-EA'-YC)$ comprising a means $(EA'-YC)$ for coupling with the first coupling element $(EA-T_3)$;

    7. a catheter (C); and

    8. a further tube $(T_4)$ connecting the catheter (C) and the second y-junction $(EA'-YC)$;

    characterised by

    9. a rigid needle carrier body (PR) which is fixed to the needle (AGO) at the end (10) of the needle nearest the common tube $(T_3)$

    10. the cap (CU) having an internal thread and being slidable over the needle;

    11. a retractable flexible covering element (GU) extending over the whole needle lenght and being connected by its ends to the needle carrier body (PR) and the cap (CU), respectively; and

    12. the means for coupling with the first coupling element of the second y-junction comprising

        12.1 two outer tongues or protuberances (40-41) adapted to engage with the thread on said cap; and

        12.2 an annular guiding member (30) for guiding the needle when the first and second coupling elements are connected.

## Revendications

1. Un appareil de dialyse continue deambulatoire dans le péritoine, comprenant:

    1. une premiére poche (SP) qui au debut est à remplir avec un liquid dialytique (LF);

    2. une deuxiéme poche qui initiallement est à vider;

    3. des tubulures de sortie ($T_1$-$T_2$) reliées à chaque poche;

    4. une première jonction ou raccord à Y ($Y_1$) réliant lesdites tubulures de sortie à une tubulure commune ($T_3$);

    5. un premier element d'accouplement (EA-$T_3$) à l'extremité libre de la tubulure commune ($T_3$) comprenant un bouchon (CU) et une aiguille (AGO);

    6. un deuxième raccord à Y ($Y_2$-EA'-YC) comprenant un moyen (EA'-YC) pour l'association au premier element d'accouplement (EA-$T_3$);

    7. un cathéter (C); et

    8. un autre tube ($T_4$) reliant le cathéter (C) et le deuxième raccord à Y (EA'-YC);

characterisé par:

    9. un corp rigide d'aiguille (PR) lequel est fixé à l'aiguille (AGO) à l'extremité (10) d'aiguille voisine à la tubulure commune ($T_3$);

    10. le bouchon (CU) ayant un filetage interne et étant coulisant sur l'aiguille;

    11. un élément de couverture flexible rétractile (GU) lequel s'entend sur toute la longueur de l'aiguille et est relié par ses extremités au corps portant l'aiguille (PR) respectivement au bouchon (CU); et

    12. le moyen pour l'association avec le premier element d'accouplement du second raccord comprenant

        12.1 duex autres langues ou protuberances (40-41) adaptes à l'engagement avec le filetage sur ledit bouchon; et

        12.2 un membre annulaire de direction (30) pour guider l'aiguille lorsque les premier et deuxième elements d'accouplement sont reliés.

**Patentansprüche**

1. Eine Vorrictung zur kontinuierlichen ambulanten Dialyse im Bauchfell, welche folgendes enthält:

    1. einen ersten Behälter oder Beutel (SP), welcher am Anfang mit dialyticher Flüssigkeit (LF) einzufüllen ist,

    2. einen zweiten Behälter (Sn) welcher am anfang zu entleeren ist;

    3. Ausgangs-röhre (T1-T2) welche an jeden Beutel Verbunden sind;

    4. einen ersten Y-Anschluss (Y1) zur Verbindung der Ausgangsröhren zu einem gemeinsamen Rohr;

    5. ein erstes Kupplungselement (EA-T3) welches am freie eEde des gemeinsamen Rohr (T3) ist und einen Deckel der Verschlucs (CU) und eine Nadel (AGO) enthält;

    6. einen zweiten Y-Anschluss (Y2-EA'-YC) welcher ein Mittel zur Kupplung mit genanntem ersten Kupplungs-element (EA-T3) aufweist;

    7. einen Katheter (C); und

    8. einen weiteren Rohr (T4) zur Verbindung des Katheters (C) mit dem zweiten Y-Anschluss (EA'-YC);

und gekennzeichnet ist durch:

    9. einem steifen Körper (PR) welcher die Nadel trägt und an gennanter Nadel (AGO) das näheresten End des gemeinsamen Rohre (T3) festgestellt ist;

    10. dem Deckel (CU) welcher mit einem inneren Gewinde Vorgesehen ist und über die Nadel gleitend ist;

    11. einem einziehbaren, flexiblen Deckungselement (GU) welches auf die ganze Nadelslänge erstreckt und an den Ende mit genannten nadeltragende Körper (PR) bzw mit dem Verschluss verbunden ist, und

    12. wobei das gennante Mittel zur Kupplung mit dem ersten Kupplungs element des zweiten Y-Anschluss enthält

        12.1 zwei äusseren Zungen oder Vorsprünge (40-41) welche im Stand sind, sich mit der in dem Deckel geformte Gewinde zu binden, und

        12.2 einen ringförmiges Glied (30) zur Führung der Nadel wann die ersteund zweite Kupplungselemente verbunden sind.

Fig. 1

Fig. 1'

Fig. 2

Fig. 3

Fig. 4